Europäisches Patentamt

(19) **European Patent Office**

**Office européen des brevets**

(11) Publication number: **0 102 807**

**A1**

(12) # EUROPEAN PATENT APPLICATION

(21) Application number: **83304880.4**

(22) Date of filing: **24.08.83**

(51) Int. Cl.³: **A 63 C 11/00**
**A 61 F 5/01**

(30) Priority: **25.08.82 US 411404**
**11.08.83 US 521965**

(43) Date of publication of application:
**14.03.84 Bulletin 84/11**

(84) Designated Contracting States:
**AT BE CH DE FR GB IT·LI LU NL SE**

(71) Applicant: **BIO-MEC SKI INDUSTRIES INC.**
**445 Veit Road**
**Huntingdon Valley, Pa. 19006(US)**

(72) Inventor: **Waddell, Thomas P.**
**1306 Broadview West**
**Downingtown Pennsylvania 19335(US)**

(72) Inventor: **Renzetti, Robin**
**2985 Hibernia Road**
**Coatesville Pennsylvania 19320(US)**

(74) Representative: **Shaw, Laurence**
**George House George Road**
**Edgbaston Birmingham B15 1PG(GB)**

(54) **Leg brace for skiers and the like.**

(57) A brace for releasable securement to at least one leg of a person includes an upper support (12) adapted to be positioned above the knee and including an upper end (20) adapted to be located immediately adjacent the buttocks-thigh interface to form a seat, and a second, lower support (36) adapted to be located beneath the knee and having a lower end (38) adjacent the foot. A flexure assembly (30, 32) is located in the region of the knee and interconnects the first (12) and second (36) supports. The flexure assembly (30, 32) enables the person wearing the system to freely bend the knee through an angular range up to a predetermined angle at which a resistance exists to further flexure. Preferably the brace includes an energy dampening section for establishing a resistance to movement of the flexure assembly (30, 32) beyond the predetermined angle to permit damped flexure of the flexure assembly (30, 32) beyond the predetermined angle to thereby absorb energy imparted to the support system (10) by the person wearing it.

./...

FIG. 2

## LEG BRACE FOR SKIERS AND THE LIKE

This invention relates to a leg brace for skiers and other active people and generally to support structures, and more particularly to systems for supporting the body weight of a person to allow various recreational and/or vocational activities.

Various types of leg braces are known. United States Patent No. 3,928, 872 (Johnson) discloses a leg brace arranged to provide flexural knee and leg support to a skier. A spring extends between support sleeves positioned above and below the knee to reduce weight load on the knees, absorb shock and reduce the strain when the knee flexes. The spring however produces an oscillatory or bouncing effect on the body as the angle of knee flexure changes during a run over the uneven terrain of a typical ski slope. The more rigid the spring, the more pronounced the rebounding effect will be, and the frequency of spring movement will be slower than the frequency of flexure of the knee as caused by the traversal of a skier over the terrain.

According to one aspect of the invention there is provided a
leg brace for use by skiers and like active people
comprising, a first support portion to be located below the
knee and an second support portion to be located above the
knee, bridging means joining the support portions,
characterised in that the support portions are joined by
flexure means arranged to enable the user to bend the knee
through an angular range up to a predetermined angle at
which there is resistance to further flexure.

Preferably the flexure means includes adjustable means for
establishing the angular range of flexure.

Preferably the flexure means comprise plural elements
coupled together by tensioning means and arranged to pivot
with respect to one another to a degree determined by the
tensioning means.

Preferably the adjustment means comprises quick acting means
wherein once the knee is flexed to the desired angle, the
quick acting means can be engaged to impede flexure beyond
that angle.

Preferably energy dampening means for establishing a
resistance to movement of the flexure means beyond the
predetermined angle and for permitting dampened flexure
beyond the predetermined angle to absorb shocks transmitted

to the body of the wearer.

Preferably the brace is incorporated in a garment.

In order that the invention may be well understood, it will now be described with reference to the accompanying diagrammatic drawings, in which

Figure 1 is a side elevation of a skier wearing a leg brace of the the invention;

Figure 2 is an enlarged perspective view of the brace of Figure 1;

Figure 2A is an enlarged isometric view of the connecting plate of the brace;

Figure 3 is an enlarged fragmentary section taken along line 3-3 of Figure 2;

Figure 4 is an enlarged section taken along line 4-4 of Figure 3;

Figure 5 is an enlarged fragmentary side elevation of the brace in a knee bending condition;

Figure 6 is an enlarged perspective view of one slug;

Figure 7 is an enlarged fragmentary section taken along line 7-7 of Figure 2;

Figure 8 is an enlarged section taken along line 8-8 of Figure 7;

Figure 9 is an enlarged section similar to Figure 8, but showing the elements oriented in a different position;

and

Figure 10 is an enlarged exploded isometric view showing the quick release mechanism.

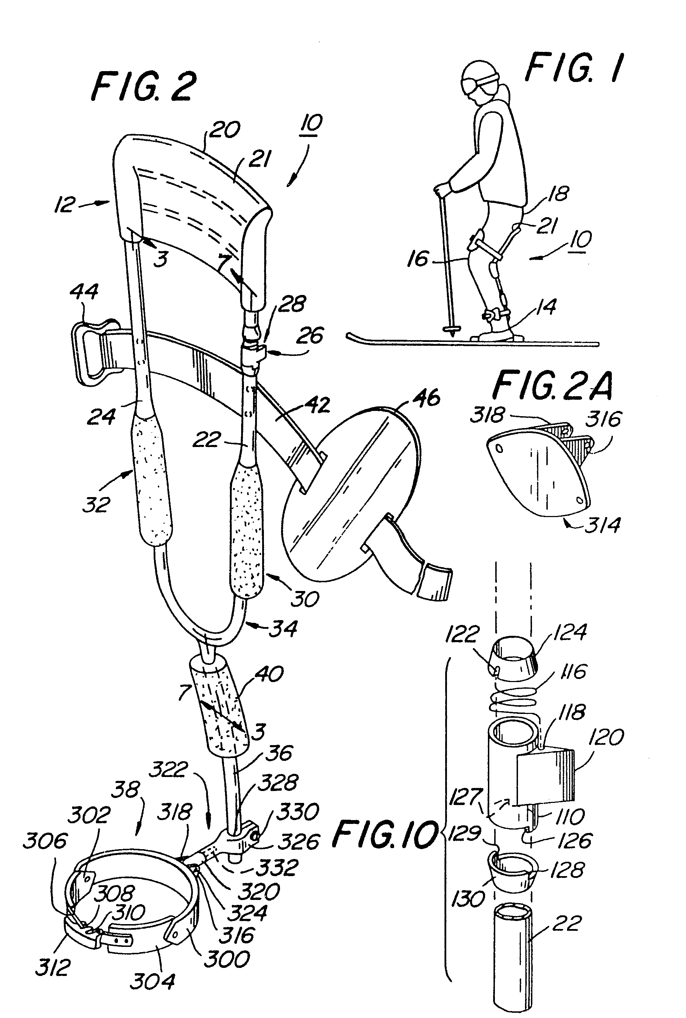

As shown in Figure 1, a leg brace comprising a body weight support system 10 comprises a brace 12 secured to a leg and ski boot 14 worn by the skier.

As shown in Figure 2, the brace 12 comprises a pair of upper tubular frame sections 22 and 24, above a generally U-shaped tubular bridging section 34 on top of a rod-like base section 36. The upper sections 22 and 24 are connected to the bridging section 34 by flexure assemblies 30 and 32, respectively. The brace is arranged so that when it is secured to the skier's leg the flexure assemblies are disposed behind the knee and towards each side of the leg. An injection molded plastic thigh support 20 bridges the frame sections 22 and 24. The support 20 has a generally concave inner curvature to conform to the general shape of the thigh area, a rounded upper-most end, and a flared back to provide a smooth surface 21 located adjacent the thigh-buttocks interface, to thereby form a seat.

The upper frame section 22, intended to be located adjacent the outside surface of the user's leg includes a quick-adjustment system 26, arranged to enable the user to preset the angle of flexure of the knee, before increased resistance to further flexure occurs. The frame section 22 includes an actuating mechanism 28, and will be described in detail below.

The upper section 24, intended to be located adjacent the medial side (inside) of the leg, houses a set of springs stacked in a column and a hydraulic dampening system, and will be described in detail below.

The rod-like base 36 has a slight concave curvature to conform to the leg of the wearer and is connected at its lower end to a ski boot attaching member 38. A cushioning pad 40 is secured about the vertical support section 36 to provide protection for the shin area when a skier crosses his legs.

The member 38 includes a rear arcuate segment 300 adapted to encircle the rear of a ski boot 14 and having a pair of front straps 302 and 304 to be secured about the front of the boot. The securing mechanism can be of any desired construction, and as shown strap 302 has a conventional link 306 positionable within a desired groove 308 or 310 of a clasp 312 pivotably secured to the other strap 304.

As can be seen best in Figure 2A a mounting plate 314 is secured by suitable fasteners (not shown) to the rear acruate segment 300. A pair of rearwardly projecting flanges 316 and 318 pivotably secure the rear arcuate segment 300 to a forward section 320 of a connecting link 322 through a laterally disposed pivot pin 324. A rear section 326 of the connecting link 322 includes a central passage 328 for receiving the vertical support rod 36 of the brace 12 and the rod is held in position by the use of a tightening screw 330.

As shown, the forward section 320 of the connecting link 322 is rotatably joined to the rear section 326 via an elongate axle 332 to permit rotational movement of the rear section 326 relative to the ski boot attaching member 38 so that substantially the entire brace 12 can be laid on its side as the member 38 is secured to the skiers boot.

The pivot connection provided by the laterally disposed pivot pin 324 permits the skier to move forwardly and backwardly relative to his ski boots to assume a desired attack position. Moreover, if the skier leans backward to a position in which his or her buttocks is not positioned over the ski boots little or no support will be provided by the brace 12. When this occurs the skier will know that he or she is in a poor skiing posture, thereby prompting the skier to change his or her body position.

The swivel movement permitted between the forward section 320 and rear section 326 of the connecting link 322 permits lateral movement of the skier's body during a ski run while the arrangement of the flexure assemblies 30 and 32 acts to preclude excessive lateral movement of the skier's knee relative to the rest of his leg, and also undesired twisting of the knee. Thus, lateral mobility of the skier's body is permitted while at the same time undesired lateral, or twisting movement of the knee is precluded.

A mounting strap 42 for securing the brace to the leg is riveted to the upper frame sections 22 and 24 and includes a buckle 44 at one end thereof; and the opposite end is adapted to receive a plate 46 thereon. The plate 46-is a flexible molded member adapted to contour the leg to spread the strap load over a wide surface to thereby avoid an undesired concentration of forces on the wearer's leg.

The quick adjustment system 26 is arranged to permit presetting of the angle through which the flexure assemblies 30, 32 can freely move before the user experiences any resistance to additional angular movement.

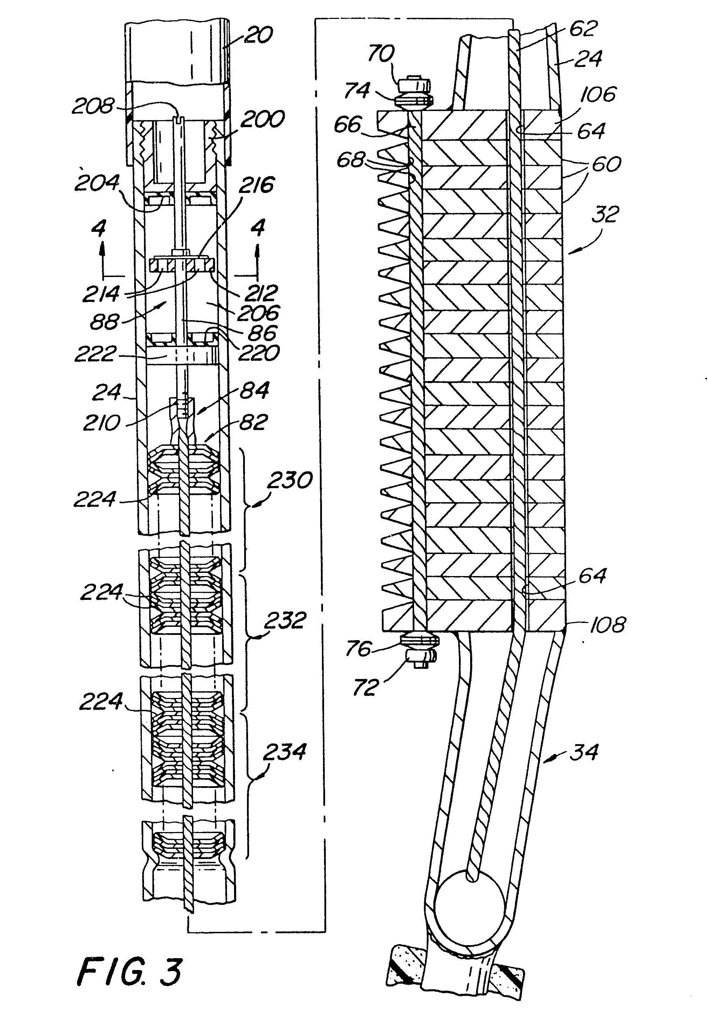

As shown in Figures 3 and 7, each flexure assemblies 30, 32 includes a stack of plate-like slugs 60, a power cable 62 extending through aligned passageways 64 adjacent the forward end of the stack (i.e., the end adjacent to the wearer's knee) and a taut alignment cable 66 extending through aligned passageways 68 adjacent the rear of the stack (i.e., the end remote from the wearer's knee). The
alignment cable 66 is maintained taut by connectors 70 and
72 swaged to the ends of the cable with disc springs 74 and
76 positioned between the connectors and the end slugs in
the stack. The adjustment system 26 controls the slack of
the power cable 62 to preset the angle through which the
flexure assemblies 30 and 32 can freely pivot.

As best shown in Figure 7  the power cable 62 extends
through an elongate sleeve 78 and compression spring 80
located within the upper frame section 22. Both this sleeve
and the spring form part of the adjustment system 26. The
power cable 62 continues through the aligned passageways 64
(Figure 3) adjacent the forward end of the stack of slugs 60
of the assembly 30. The cable continues through the hollow
interior of the bridging section 34, and then upwardly
through the aligned passageways 64 at the forward end of the
stack of slugs 60 of the flexure assembly 32 (Figure 3).
The cable then passes into the hollow upper support rod 24,
and then through a dampening spring set 82 mounted therein.
The upper end of the cable is swaged to the lower end of a
connector 84, the upper end of which is threaded to receive
the threaded lower end of a piston rod 86 forming part of a
hydraulic dampening system 88, to be described in detail
below.

From the above description it should be apparent that the amount of slack established in the power cable 62 determines
or sets the angle through which the slugs 60 in each of the
flexure assemblies 30 and 32 can be freely moved without
encountering substantial resistance.  Once the preset
angular movement of the assemblies has taken place the power
cable 62 becomes taut, and further angular movement is
resisted by the dampening spring set 82 in a manner to be
described in detail below.

As best shown in Figure 7, the system 26 includes an
elongate sleeve 78 including longitudinally spaced apart,
arcuate annular recesses 100 to receive locking ball
bearings 102 and also to engage within openings (to be
described later) in the wall of the upper frame section 22
to retain positively the sleeve 78 in a fixed longitudinal
position relative to said section.  In order to permit
adjustment of the sleeve 78 relative to the upper support
section 22 the ball bearings 102 are permitted to withdraw
from the annular recesses 100 as described below.

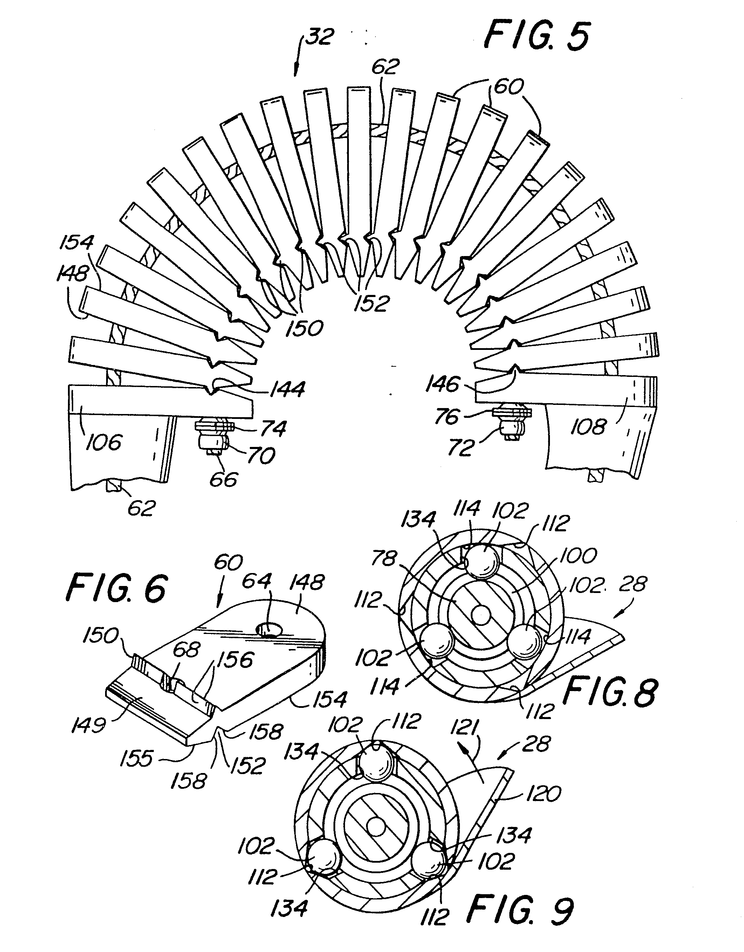

As shown in Figure 8, three position-locking ball bearings
102 are located about the periphery of one of the annular
recesses 100.  A similar set of three ball bearings is
positioned in an annular recess located below the recess
shown in Figure 8.  Only one ball bearing of each set is
illustrated in Figure 7.  Although six ball bearings are
utilized in the preferred embodiment of the invention, the specific number and location of these ball bearings can be varied.

As shown in Figure 7, the compression spring 80 is positioned between the base of the elongate sleeve 78 and the upper surface of an upper support pad 106 of the flexure assembly 30. This compression spring imparts an upward force to the sleeve 78, as viewed in Figure 7. This upward force causes the ball bearings 102 to move out of their respective recesses when said ball bearings are free to move in a radially outward direction, thereby permitting the sleeve 78 to move upwardly. Upward movement of the sleeve 78 maintains the cable 62 taut.

The actuating mechanism 28 is shown in Figures 8 to 10. This mechanism operates to permit the ball bearings 102 to move radially outwardly from their respective annular recesses 100 to permit adjustment of the predetermined angular range through which the flexure assemblies 30 and 32 can freely move. The adjustment mechanism 28 includes a collar 110 rotatably secured about the outer periphery of the frame section 22 (Figure 7). A plurality of tapered recesses 112 is machined into the inside periphery of the collar, and is spaced-apart to align with the three annularly spaced-apart ball bearings 102 in each set of said ball bearings.

Figure 8 showns the collar 110 at rest with the recesses 112 being out of radial alignment with the ball bearings 102. In this position inner surface regions 114 of the collar between the spaced-apart recesses 112 provide obstructing surfaces to prevent ball bearings from moving radially out of the annular recesses 100 of the elongate sleeve 78. Thus, in the normal position of the collar 110 the sleeve 78 is fixed within the upper frame section 22 by the connection between the sleeve and frame section provided by the ball bearings.

As shown in Figure 10, one end of a torsion spring 116 is positioned within a slot 118 located in a tab or button 120 of the collar 110. This tab is engaged by the skier for the purpose of rotating the collar in the direction indicated by arrow 121 in Figure 9. The opposite end of the torsion spring is engaged within a slot 122 of an upper retaining cap 124 which preferably is welded to the outer periphery of the frame section 22. This torsion spring 116 normally biases the collar 110 into the position illustrated in Figure 8 to retain the ball bearings 102 in their respective annular recesses 100. The position of the collar indicated in Figure 8 is maintained by the co-operation between a downwardly projecting ledge 126 of the collar and an upwardly projecting ledge 128 of a lower retaining cap 130. This latter retaining cap, like the retaining cap 124, preferably is welded to the outer periphery of the frame

section 22.

Referring specifically to Figure 9 the actuating tab 120 is
pressed in the direction indicated by the arrow 121 to
rotate the collar 110 against the torsion spring 116 to
align the recesses 112 with respective ball bearings 102.
The ball bearings are forced out of these recesses by the
upward linear movement of the sleeve 78 caused by the
compression spring 80. Rotation of the collar 110 in the
direction of arrow 121 is limited by the co-operation of a
downwardly projecting ledge 127 of the collar with an
upwardly projecting ledge 129 of the lower retaining cap
130.

With the collar 110 in the orientation illustrated in Figure
9, the compression spring 80 acts to drive the sleeve 78 to
its uppermost setting, i.e., the lower set of ball bearings
102 is in the lowermost groove 100. With the collar 110
being manually maintained in the sleeve release position
illustrated in Figure 9 the skier can then bend his or her
knee to a predetermined angle, representing the angle the
skier desires to assume when skiing. As the skier bends at
the knees the sleeve 78 will be forced downwardly against
the opposing force of the compression spring 80 to permit
the slugs 60 to pivot with respect to each other while still
maintaining the cable 62 taut. When the skier reaches a
desired position the tab 120 is released to cause the collar 110 to be rotated by torsion spring 116 back to the position
illustrated in Figure 8, thereby forcing the ball bearings
102 into aligned annular recesses 100 of the sleeve. This
sets the angle through which the knees can freely bend
without encountering substantial resistance.

It should be noted that the various ball bearings 102 extend
into aligned passages 134 in the wall of the upper frame
section 22. The ball bearings 102 are prevented from
falling out of the support section 22 by the collar 110
which closes the passages. The collar 110 can include one
or more enlarged openings (not shown) which can be aligned
with the openings 134 in the upper frame section 22 for the
purpose of initially inserting the ball bearings 102 into
the interior of said rod. After the ball bearings have been
inserted into the frame section 22 the collar 110 is located
and maintained in a position to prevent the openings in it,
through which the ball bearings initially were loaded, from
becoming aligned with the spaced-apart passages 134 through
the frame section 22. This prevents the individual ball
bearings 102 from falling out of the upper frame section 22.

In the preferred embodiment of this invention the
constructional arrangement of the flexure assemblies 30 and
32 are identical. As shown in Figure 3, the upper support
pad 106 is welded to the lower end of the upper support
frame section 24 to provide an interface with the slugs 60 and also to support one end of the compression spring 80. A similar lower support pad 108 is welded to the upper end of one of the legs of the U-shaped lower bridging section 34 to likewise provide an interface with the slugs 60. As can be seen best in Figure 5 the surface of the upper support pad 106 engaging an adjacent slug 60 includes a recess 144 formed in it, and the surface of the lower support pad 108 engaging the lowermost slug 60 includes a ridge 146 in it.

The recess 144 is identical to the recesses provided in each of the slugs 60, and the ridge 146 is identical to the ridges provided in each of the said slugs 60.

The detailed construction of one of the slugs 60 is shown in Figure 6. This slug includes an elongate passageway 64 adjacent the forward end thereof (the end adjacent the skier's knee) and a centrally located passageway 68 located adjacent the rear end thereof. The upper surface 148 of each slug includes a lateral ridge 150 which is interrupted in the middle thereof by the passageway 68. A lateral recess 152 is provided in the bottom surface 154 of the slug in vertical alignment with the lateral ridge 150 on the adjacent upper surface 148.

As shown in Figures 3 and 7, segments 149 and 155 of the upper and lower surfaces 148 and 154, respectively, are located rearwardly of the vertically aligned ridge 150 and recess 152 of each slug and are inclined to co-operate with corresponding inclined segments of adjacent slugs to define a back taper angle between the adjacent slugs 60 of the flexure assemblies 30 and 32. This back-taper angle permits rotational movement of the slugs 60 relative to each other about the fulcrum established by the engagement of the laterally extending ridges 150 of said slugs with the recesses 152 of adjacent slugs, as is illustrated best in Figure 5.

An extremely important feature of this invention is that the interaction of the laterally extending ridges 150 with adjacent laterally extending recesses 152 wholly or - substantially prevents rotational twisting of the slugs relative to each other. This prevents failure of the flexure assemblies 30 and 32, and also helps to absorb and reduce twisting loads generally imposed upon the human knee joints, particularly when a skier falls. The taut adjustment cable 66 provides a beneficial function in maintaining the apex of each ridge 150 seated against the base of its co-operating recess 152 to assist in preventing the rotational twisting movement between the adjacent slugs 60 of the flexure assemblies.

In order to prevent undesired wear of the inclined surfaces 156 of the ridges 150 and the inclined surfaces 158 of the recesses 152 it is desirable to design the slugs so that they can move relative to each other through their full angular range, without the inclined surfaces 156 and 158
rubbing excessively against each other.  To accomplish this
result it is preferred that the recess angle (the included
angle  between the inclined surfaces 158 of the recess)
equal the sum of the ridge angle (the included angle between
the inclined surfaces 156 of the ridge) and the back taper
angle (the included angle between adjacent inclined rear
segments 149 and 155 of the top and bottom surfaces of
adjacent slugs).  The recess angle preferably should be no
less than the sum of the ridge angle and the back taper
angle, and preferably should be no more than 10% greater
than said sum.

In operation, the back taper angle determines the maximum
angular movement that can be achieved between the adjacent
slugs 60.  Because the recess angle is at least as great as
the sum of the back taper angle and the ridge angle, the
inclined surfaces of the ridge will not engage the inclined
surfaces of the recesses throughout the full range of
angular movement permitted by the back taper angle.
Accordingly, very little wear occurs at the fulcrum
established between adjacent slugs.  The taut alignment
cable 66 provides uniform flexure of the flexure assemblies
30 and 32 by maintaining the ridges 150 in proper seated
position within co-operating recesses 152.

The quick-acting adjustment system 26 permits the setting of a reference point for the flexure assemblies 30 and 32 to move through a predetermined angle. The flexure assemblies are thus free to move through the desired angular position up to the support point without any substantial resistance to flexure. Thereafter, the power cable becomes taut and acts through a dampening spring set 82 to assist in absorbing excessive energy imposed on the brace 12 by the skier.

The dampening spring set 82 is designed to provide a varying resistance to flexure of the flexure assemblies 30 and 32 beyond the set point, thereby to absorb excessive energy imposed on the brace 12 by the human body as the skier is moving over rough terrain. In particular, the spring set permits the brace 12 to absorb the bumps by permitting the skier's legs to continually bend beyond the set point against a dampening resistive force imposed by the spring net.

The brace 12 also includes a hydraulic system, to be described below, to dampen the spring rate of the spring set 82 in a direction tending to urge the skier back to the set point, after flexure beyond the set point has occurred. The brace 12 basically includes a fluid actuated shock absorbing system so that the spring rate of the spring set 82 is controlled, or dampened to permit the skier to come back easily to the support point, thereby eliminating the bounciness that otherwise might occur.

As shown in Figure 3 the interior surface of the upper frame
section 24 is internally threaded to receive a threaded plug
200 therein. The shaft 86 passes through an opening in the
base of plug 200, and then through an oil seal 204
positioned adjacent the base of said plug. This oil seal
which can be a conventional spring loaded TEFLON seal, such
as that sold by Bal Seal Engineering Company, 620 W Warner
Avenue, Santa Anne, California 92707, seals against the
inner periphery of the support rod 24 to provide an upper
seal for a chamber 206 in which hydraulic fluid, e.g., oil
is retained. The base of the hydraulic fluid chamber 206 is
provided by a lower oil seal 220 which can be a spring
loaded seal identical to the upper seal 204. The seal 220
sits on top of a substantially flat plate 222 welded to the
inner periphery of the frame section 24. Thus, the lower
oil seal 220, together with the upper oil seal 204 and the
inner periphery of the upper frame section 24, define the
hydraulic oil chamber 206.

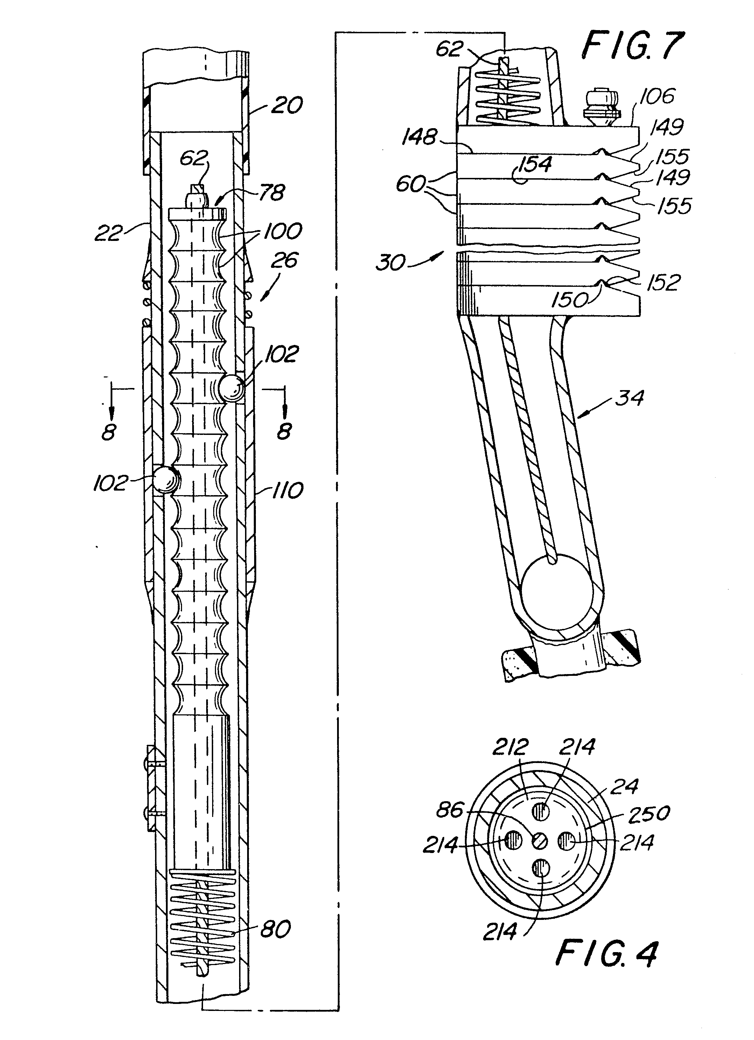

As shown in Figure 3 and 4, the upper end of the shaft 86 is
provided with a screwdriver slot 208 to permit the lower
threaded end 210 of the shaft to be secured within the upper
threaded section of the connector 84. A piston 212 is
secured to the shaft 86 and includes a plurality of passages
214 extending through it, four such passages being shown.

A check valve 216, comprising a thin metal washer, is located above the piston 212 and is adapted to close off the passages 214 to the movement of hydraulic fluid from above the piston 212 to a position below the piston.

Referring specifically to Figure 3, the dampening spring set 82 is stacked in a column about 15 cm high. This set includes approximately 160 disk-type springs 224, such as Belleville springs adapted to deflect under a predetermined load. Suitable Belleville springs are sold by Rolex Company, 385 Hillside Avenue, Hillside, New Jersey 07275.

As shown, the spring set 82 is divided into three discrete sections 230, 232 and 234, to provide progressively greater resistance to bending. Each section is approximately 5 cm high. In the first set 230 nested pairs of springs (i.e. tow springs in parallel) are stacked in series and each pair has a maximum deflection rated at 660 kilograms. The middle section 232 includes nested triples (i.e. three springs in parallel) rated at 990 kilograms and being stacked in series. The third section 234 includes nested quads (i.e. four springs in parallel) having a maximum deflection rated at 1320 kilograms and being stacked in series.

An analysis of dynamic loading indicates that a maximum size load of 114 to 228 kilograms generally is imposed upon the spring set 82, depending upon the weight of the skier and the angle of knee bend determined by the set point of the
flexure assemblies 30 and 32.  Thus, the spring set may be
partially depressed to support this load.

When a skier hits a bump, such as when skiing on rough
terrain, a maximum dynamic load of as much as 5 G's (570 to
1140 kilograms) can be imposed upon the brace 12.  Under
these conditions the springs will gradually collapse or
deflect to absorb this dynamic loading.

The deflection of the spring set thus allows for additional
knee bending beyond the predetermined set point, to permit
the gradual dissipation of the shock which otherwise would
be abruptly absorbed by the skier's body.

In the preferred system of this invention the spring set 82
is designed to deflect a maximum of approximately 2.5 cm,
thereby providing for 2.5 cm of movement of the power cable
62.  This equates to approximately 40° of angular movement
of the flexure assemblies 30 and 32 to thereby permit a
corresponding additional angular movement of the knees of
the skier beyond the set point.

Unimpeded or unrestricted downward movement of the power
cable 62 is permitted by the provision of the passages 214
through the piston 212 as the springs 224 deflect.
Specifically, during this downward movement the check valve

0102807

216 is open to permit the substantial unimpeded flow of hydraulic fluid through these passages. This prevents the high viscosity of the fluid from excessively impeding or retarding the downward movement of the power cable 62 as the individual disk springs 224 deflect under dynamic loading. However, upward movement of the power cable 64 to reposition the flexure assemblies 30 and 32 back to the desired set point is retarded or dampened by the inclusion of the check valve 216 which precludes hydraulic fluid from passing through the passages 214. With these passages closed the hydraulic fluid is only permitted to move slowly through a small restrictive annular passage 250 established between the side peripheral edge of the piston 212 and the inner periphery of the support section 24 (Figure 4). In a preferred embodiment of this invention the cycle time from the bottom of the stroke (with the individual springs 224 deflected under dynamic loading) to full return to the preset point is approximately one second, and the hydraulic cylinder pressure is in the range of 7,000 pounds per square inch.

The provision of sections in the spring set 82 having different maximum deflection ratings provides for the progressive absorption of energy imparted to the body weight support system under the dynamic conditions encountered by a skier moving over rough terrain. Only some of the springs will deflect or collapse at the same time under dynamic loading conditions, and the spring will gradually collapse to gradually absorb the energy.  Although a specific arrangement for stacking the springs in three distinct sections has been disclosed, it should be noted that the springs can be nested in various different arrangements to provide the desired load responsive characteristics in the spring set 82.

The ski brace can be incorporated into a garment, such as a pair of ski pants.  Each leg of the garment includes a pair of longitudinally extending passageways formed by sewing respective fabric strips onto the inside of each leg.  Each of these passageways is arranged to receive therein a respective upper frame section (i.e. 22, 24) flexure assembly (i.e. 30, 32) and spaced apart upper portions of the legs forming the U-shaped bridging section 34.  The legs of the bridging section 34 extend through respective openings in each of the passageways, which can be openings provided in the fabric strips, so that the lower portion of the brace is exposed within the interior of the pants' leg for ready connection to the ski boot.

The tab 120 of the adjustment means can be positioned to extend through an opening in the outer layer of the garment to permit easy access for adjusting the angle through which the flexure assemblies 30 and 32 are free to bend without encountering significant resistance.

The adjustment of the degree of flexure of the angle enables skiers readily to set the point at which the knee bend angle produces total support for the body, thereby resulting in virtually weightless skiing.  This relieves leg strain and tends to centre the skier's weight over the skis which is desirable for effective skiing.  The energy dampening system of this invention provides a damped supportive force which does not oscillate or rebound, thereby insuring that the skier can maintain effective control, while at the same time absorbing shocks imposed on the skier's body.  The top-fitting plastic thigh support member 20 spreads the body weight over a large surface on the rear side of the upper leg so that there are no uncomfortable pressure points. Because of the attachment to the boots, most of the body weight is transferred to the boots.  Thus, the skier can "sit" on the system while remaining in good attack posture. The resulting relaxation of the leg muscles during such operation eliminates the normal muscle strain and fatigue associated with skiing, thereby making skiing a much more pleasurable recreation.

The unique arrangement and construction of the slugs 60 employed in the flexure assemblies tends to prevent the imposition of undesired torsional stresses on the skier's leg, while at the same time permitting normal bending of the skier's knee.

The location of the laterally spaced-apart support means halfway between the rear and side of the leg puts the support means out of the way during a fall, since during most falls the skier either lands on the rear portion of the legs or on the side-hip area.

The system can be utilized by skiers of various heights. The adjustment feature of the support system of the instant invention enables one to accurately reset the system to any desired position, thereby compensating for any wear-induced changes which may occur over the long life of the system.

The movable connections between the member attached to the boot and the upwardly directed supports secured to the wearer's leg permits increased latitude in body movement of the skier. This connection makes the invention suitable for use in teaching and/or training applications by encouraging the skier to maintain a proper skiing posture in order to obtain the body-supporting benefits of the invention. Persons previously unable to ski due to some physical defect, such as amputees, persons with muscle or nerve disorders, bad knees, etc., can ski effectively and safely. Thus, the instant invention provides for safer, more aggressive and effective skiing for all persons.

The leg brace including a support system has a wide range of applications beyond skiing. The system can be used whenever applications beyond skiing.  The system can be used whenever it is desirable to provide support for a person who must stand with the knees bent in a predetermined position for an extended period of time.  One example of such applications is use by farm workers for manual picking of low growing crops, inasmuch as the system can provide capability for total support of the body while allowing the wearer to move in a normal manner.

CLAIMS

1. A leg brace for use by skiers and like active people comprising a first support portion to be located below the knee and a second support portion to be located above the knee, bridging means joining the support portions, characterised in that the support portions are joined by flexure means arranged to enable the user to bend the knee through an angular range up to a predetermined angle at which there is resistance to further flexure.

2. A brace according to Claim 1 characterised in that the flexure means includes adjustable means for establishing the angular range of flexure.

3. A brace according to Claim 1 or 2, characterised in that the flexure means comprise plural elements coupled together by tensioning means and arranged to pivot to one another to a degree determined by the tensioning means.

4. A brace according to Claim 3 characterised in that the tensioning means comprises an elongated, flexible cable on which the plural elements are strung.

5.   A brace according to Claim 3 or 4, characterised in that
the tensioning cable is connected between said upper and
lower portions and the flexure means includes adjustable
means for shortening or lengthening said cable means to
establish the tension in the tensioning cable.

6.   A brace according to Claim 3, 4 or 5, characterised in
that each element is tubular and includes surface means
adapted to engage the immediately adjacent element to
facilitate uniform pivoting of the elements.

7.   A brace according to any preceding Claim, characterised
in that the lower support portion includes releasable
securement for anchoring the lower support to a ski boot or
the like.

8.   A brace according to Claim 2, characterised in that the
adjustment means comprises quick acting means wherein once
the knee is flexed to the desired angle, the quick acting
means can be engaged to impede flexure beyond that angle.

9.   A brace according to any preceding Claim, characterised
in that each support means comprises a pair of generally
parallel elongated, frame-like members having webbing means
interposed therebetween for engaging an associated portion of the leg of the skier.

10. A brace according to Claim 9, <u>characterised</u> <u>in</u> <u>that</u> strap means are present for strapping the upper support to the thigh just below the buttocks and just above the knee and wherein the lower support includes strap means for strapping the lower support to the calf just below the knee.

11. A brace according to Claim 10, <u>characterised</u> <u>in</u> <u>that</u> the strap means for securing the second mounting means to the boot is adjustably mounted on the lower support to provide an optimal interface with said boot.

12. A brace according to Claim 10 or 11, <u>characterised</u> <u>in</u> <u>that</u> the flexure means includes adjustment means located adjacent the upper end of the upper support for establishing the predetermined angular flexure range.

13. A brace according to any preceding Claim, <u>characterised</u> <u>by</u> energy dampening means for establishing a resistance to movement of the flexure means beyond the predetermined angle and for permitting dampened flexure beyond the predetermined angle to absorb shocks transmitted to the body of the wearer.

14. A brace according to Claim 13, <u>characterised</u> <u>in</u> <u>that</u> said energy dampening means includes spring means for deflecting under loading exceeding a predetermined level.

15.  A brace according to Claim 13 or 14, characterised in that the spring means includes a set of spring elements stacked in a column.

16.  A brace according to Claim 15, characterised in that the set of spring elements includes sections with different maximum deflection ratings for permitting gradual dampened movement of the flexure means beyond said predetermined angle.

17.  A brace according to Claim 16, characterised in that the sections of the set of spring elements having different maximum deflection ratings include differing numbers of discs nested in parallel.

18.  A brace according to Claim 13 to 17, characterised by additional dampening means for assisting in controlling the rate of movement of the energy dampening means in a direction returning the flexure means to said predetermined angle.

19.  A brace according to Claim 18, characterised in that said additional dampening means is a fluid-actuated dampening system.

20.  A brace according to any of Claims 13 to 19, characterised by connecting means joining the cable to an additional dampening means for assisting in controlling the rate of movement of the stack of discs in a direction opposed to the direction of movement of the stack during dampened flexure of the flexure means.

21.  A brace according to Claim 20, characterised in that the additional dampening means includes an elongate rod secured to the connecting means and extending through a chamber in which hydraulic fluid is retained, and a piston secured to said rod within the chamber for co-operating with the hydraulic fluid to dampen the movement of the stack of springs in a direction opposed to the direction of movement of the stack during dampened flexure of the flexure means.

22.  A brace according to Claim 21, characterised by the provision of passages through the piston and a movable valve means overlying the passages for permitting hydraulic fluid to pass through the passages as the spring elements are being deflected to provide dampened flexure of the flexure means and for preventing hydraulic fluid from passing through the passages during movement of the spring elements in a direction opposed to the deflecting direction of movement of the spring elements to dampen the return movement of the set of spring elements from a deflected state.

23.  A brace according to any of Claims 13 to 22, characterised in that the elements are substantially flat members stacked upon each other and including co-operating fulcrum means between adjacent members about which the members can pivot with respect to one another.

24.  A brace according to Claim 23, characterised in that the fulcrum means includes a ridge of one member engaging within a co-operating recess of an adjacent member, the ridge and recess associated with adjacent members extending generally transverse to the plane of flexure of the members.

25  A brace according to Claim 24, characterised in that each of the elements includes a forward end adapted to be located adjacent the knee of the user and a rearward end adapted to be located remote from said knee, opposite surface regions of adjacent elements located rearwardly of the fulcrum means being inclined away from each other to provide a back taper angle for permitting pivotable movement of the elements about the fulcrum means.

26.  A brace according to Claim 24 or 25, characterised in that one surface of each element includes an elongate ridge and the opposed surface includes an elongate recess, the ridge and recess of each element being vertically aligned with each other and being interrupted by a passageway extending through said element, the passageways of adjacent
elements being aligned for receiving a tensioning means to
maintain the transversely extending, confronting ridge and
recess of adjacent elements in engagement with each other as
the elements are pivoted with respect to one another.

27.  A brace according to any of Claims 13 to 26,
characterised in that the transversely extending recesses
include inclined surfaces and the transversely extending
ridge include inclined surfaces, the angle defined by the
inclined surfaces of the recesses being no less than the sum
of the back taper angle between adjacent elements and the
included angle between the inclined surfaces of the ridges.

28.  A brace according to any preceding Claim, characterised
in that the upper support means includes a hollow support
rod, the adjustment means including an elongate sleeve
movably mounted within said support rod, the sleeve
including a plurality of annular recesses disposed along the
elongate dimension thereof, a plurality of recess-engaging
members maintained in a fixed position within the hollow
support rod for engaging within selected annular recesses to
maintain the sleeve in a desired position relative to the
rod, and means for permitting release of the recess-engaging
members from the annular recesses to permit movement of the
elongate sleeve within said rod to thereby adjust the
tension in the cable.

29.  A brace according to Claim 28, <u>characterised</u> <u>in</u> <u>that</u> the recess engaging members are ball bearings engaged within openings in the hollow support rod, the means for permitting release of ball bearings including a collar rotatable about the outer periphery of the support rod and including recessed regions adapted to align with the openings in the support rod in which the ball bearings are retained for receiving the ball bearings as they move out of engagement with an aligned annular recess of the elongage sleeve.

30.  A brace according to any preceding Claim, incorporated in a garment.

**FIG. 2**

**FIG. 1**

**FIG. 2A**

**FIG.10**

FIG. 3

FIG. 7

20

62

78

22

100

26

102

8          8

102

110

80

62

106

148        149
154        155
60         149
           155
30

150   152

34

FIG. 4

212   214

86         24
           250
214        214

214

FIG. 5

32
62
60
154
148
150
152
144
146
74
76
70
72
66
62
106
108

FIG. 6

60
64
148
150
68
156
149
154
155
158
158
152

134  114  102
78              112
                100
112            102  28
102            114
114
112

FIG. 8

102  112
134        121  28
                120
102            134
112            102
134      112

FIG. 9

## European Patent Office

## EUROPEAN SEARCH REPORT

Application number

EP 83 30 4880

### DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| D,A | US-A-3 928 872  (JOHNSON)<br>* Abstract; figures 1-3 * | 1 | A 63 C   11/00<br>A 61 F    5/01 |
| | * Figures 4-6 * | 7,10,<br>11 | |
| | --- | | |
| A | DE-A-2 238 038  (AUE-UHLHAUSEN)<br>* Claims 1,2 * | 2,13 | |
| | --- | | |
| A | DE-A-1 578 974  (GRETSCH & CO.)<br>* Figures 3,4 * | 19,21 | |
| | --- | | |
| A | DE-A-1 478 143  (MARKER)<br>* Figure 1 * | 22 | |
| | --- | | TECHNICAL FIELDS SEARCHED (Int. Cl. ³) |
| A | EP-A-0 039 578  (DAVIS) | | |
| | --- | | A 61 F |
| A | US-A-4 136 404  (LANGE) | | A 63 B<br>A 63 C |
| | --- | | |
| A | DE-C- 838 479  (ZAPF) | | |
| | ----- | | |

The present search report has been drawn up for all claims

| Place of search | Date of completion of the search | Examiner |
|---|---|---|
| THE HAGUE | 23-11-1983 | SCHLESIER K.G.W.P. |

EPO Form 1503 03.82